# EUROPEAN PATENT APPLICATION

(11) **EP 4 781 892 A1**
(43) Date of publication of application: **29.07.2026**
(21) Application number: 25153595.1
(22) Date of filing: 23.01.2025
(51) Int. Cl.: A61B 1/00, A61B 1/04, A61B 1/07, G02B 6/00, G02B 6/06, G02B 23/26, G06N 3/02, A61B 90/00

(54) **OPTICAL SYSTEM FOR A SURGICAL DEVICE**

(71) Applicant: Quest Photonic Devices B.V., 1771 SR Wieringerwerf (NL)
(72) Inventor: MOOIJ, Joris, 1768 BN Barsingerhorn (NL)
(74) Representative: Seemann & Partner Patentanwälte mbB

(57) **Abstract**

The invention relates inter alia to an optical system for a surgical device, in particular a surgical instrument, comprising an optical unit having an optical fiber bundle with an input side and an output side, wherein the input side has at least one or more fiber bundle entrances and the output side has at least one or more fiber bundle exits, wherein the sum of the number of the fiber bundle entrances and of the number of the fiber bundle exits is greater than 2.

## Description

The invention relates to an optical system for a surgical device, in particular a surgical instrument. Moreover, the invention relates to a use of an optical system and to a method for operating an optical system.

Imaging has developed into a tool which is in particular used to perform surgical procedures with a surgical instrument. Optical imaging during surgery allows to acquire more information about the patient and its current status as well as to perform precise treatment. In endoscopic or laparoscopic surgery, optical imaging using an endoscope makes it possible to visually inspect the surgical area in the first place.

To obtain images from an object, optical imaging techniques are used in a wide range of applications. In medical applications, optical imaging techniques are used with reduced invasive effects or free of invasive effects.

A significant technique of optical imaging used in the context of medical procedures is fluorescence imaging, in particular fluorescence endoscopy or fluorescence guided surgery. This technique uses specialized dyes, which are excited by at least one excitation light source, to label and highlight certain structures of a surgical area. Typically, excitation wavelengths are in the near infrared, e.g., in a range of 800 nanometers to 1100 nanometers, or in other cases in the visible spectrum, depending on the dye.

A suitable tool for acquiring simultaneous images of multiple wavebands are multi-sensor cameras. In one embodiment of a multi-sensor camera, incoming light from a single optical path is split by a prism assembly into two or more separate paths, continuing to propagate towards separate sensors.

It is an object of the invention to provide an improved optical imaging of objects in particular for surgical instruments or the like.

The object is solved by an optical system for a surgical device, in particular a surgical instrument, comprising an optical unit having an optical fiber bundle with an input side and an output side, wherein the input side has at least one or more fiber bundle entrances and the output side has at least one or more fiber bundle exits, wherein the sum of the number of the fiber bundle entrances and of the number of the fiber bundle exits is greater than 2.

According to the invention, the optical unit is provided as an optical fiber bundle beam splitting and/or combining unit. The optical fiber bundles are made out of light guide cables like fiber optics or fibers, which in turn consist of a core or a cladding, generally made out of a type of glass. The incident light of an object or the like enters at the input side via the surfaces of the fibers of the fiber bundle entrances and exits at the output side via the surfaces of the fibers of the fiber bundle exits. The light, which is transmitted through the optical unit, can be split or partially split during its transmission or can be combined or partially combined during its transmission. In particular the split and transmitted light, which is captured at the output side as images, can be unrandomized or unscrambled by an unscrambling or unrandomizing algorithm, which can be executed by a processing device, like a computer.

According to an embodiment of the invention, the optical unit is configured as a fiber bundle beam splitter or as a fiber bundle beam combiner or as a bundle beam splitter and combiner.

Preferably, the optical fiber bundle of the optical unit has an amount of a predetermined number n of individual fibers, wherein the predetermined number n of the individual fibers is divided between k (k=1, 2, 3, ...) fiber bundle entrances at the input side and/or wherein the predetermined number n of the individual fibers is divided between l (l=1, 2, 3, ...) fiber bundle exits at the output side. The fibers of the optical unit are arranged between the input side and the output side. The amount n of individual fibers is preferably equally divided between k and the same amount of n fibers is preferably equally divided, but not necessarily equally divided, between l (l = 1, 2, 3, ...) amount of fiber bundle exits. The divisions of the fibers can be provided in the same arrangement, but also in a different arrangement. An example way of arrangement of the fibers is randomly at the fiber bundle entrances and the fiber bundle exits.

According to one embodiment, the optical unit as a fiber bundle splitter comprises a fiber bundle with one fiber bundle entrance and two fiber bundle exits. The n fibers are all present at the single fiber bundle entrance and the n fibers are equally divided into two fiber bundle exits, both exists having n/2 fibers. The fibers of the optical unit are arranged randomly between the two exits. In particular the amount n of the fibers is preferably large enough to mix a small amount of light entering the fiber bundle splitter to be equally divided, despite the random arrangement of the fibers.

In another embodiment of the optical unit as a fiber bundle combiner, the optical unit comprises a fiber bundle with two fiber bundle entrances and one fiber bundle exit. The n fibers are equally divided into two fiber bundle entrances, wherein both fiber bundle entrances having n/2 fibers and the n fibers are all present at the single fiber bundle exit as only one exit. The fibers are arranged randomly between the two fiber bundle entrances. The amount n of the individual fibers is large enough to mix a small amount of light entering the exclusively one fiber bundle entrance of the fiber bundle combiner to be equally divided at the exit, despite the random arrangement of the fibers.

According to a further embodiment, the optical unit as a fiber bundle splitter and combiner comprises a fiber bundle with two fiber bundle entrances and two fiber bundle exits. The n fibers are equally divided into two entrances and two exits, all fiber bundle ends having n/2 fibers. The fibers are arranged randomly between the two fiber bundle entrances and fiber bundle exits. In particular, the amount of the n fibers is large enough to mix a small amount of light entering one or the other fiber bundle entrance of the fiber bundle splitter and combiner to be equally divided at both fiber bundle exits.

Within the scope of the invention, the three above-mentioned embodiments are understood not to limit higher number of fiber bundle entrances and/or fiber bundle exits. Furthermore, the embodiments according to the invention are not limited to other modifications such as fiber division, un-even divisions, entrance and/or exist cross-section surface shape etc., which also fall under the invention.

The optical system is further characterized in that the optical system is configured as a multispectral and/or hyperspectral camera, wherein in particular at least one objective lens is provided in front of the input side for projecting an image onto the at least one fiber bundle entrance.

In an embodiment of the optical system as a multispectral and/or hyperspectral camera comprising a fiber bundle splitter (as an optical unit) with one fiber bundle entrance and two or more fiber bundle exits, the optical unit is used as the image splitter. The fiber bundle splitter comprises at least one objective lens, e.g of a camera system or the like. Hereby, the lens projects an image of an object onto the fiber bundle entrance. Each fiber of the fiber bundle entrance collects the localised light of the projected image and transmits it to the two or more fiber bundle exits. In this embodiment of the invention, the fibers at the input side are locally sorted to one of the two or more fiber bundle exits, in particular similar to how a Bayer filter sorts or structures its filter pattern, equally distributed in the x-direction and y-direction of the image. Although the raw resolution of the image entered the fiber bundle and is split in between two or more images compared to the input, the spatial continuity of the scene and of the image are maintained. The split images at the fiber bundle exits are then projected through respective band pass filters or the like. It is to be noted that the variation of this embodiment is not limited to only band-pass filters. The images at the fiber bundle exits are projected onto one or more image sensors, like CMOS or similar sensors.

In another embodiment of an optical system having a multispectral and/or hyperspectral camera, the fibers are not sorted into one of the two or more fiber bundle exits, but the fiber are instead randomly sorted into one of the two or more fiber bundle exits. If the random sorting of the fiber is random enough, this should ensure localised light of the projected images still be split evenly across all the fiber bundle exits. In this case, all fiber bundle exits project each a scrambled image onto a corresponding image sensor. To un-scramblase or un-randomise these captured images, a , in particular one time, unscrambling or un-randomize algorithm can be employed per output of the fiber bundle exits to obtain the spatial coordinates of the fibers on the surfaces of the fiber ends with respect to the fiber on the input surface of the fibers at the fiber bundle entrances. This is possible due to the fact that the randomised order of the fibers is fixed in place in the fiber bundle or the optical unit.

Within the scope of the invention, the unscrambling method or algorithm is configured to give the multispectral and/or hyperspectral camera with the random fiber bundle one or more different object targets in front of the at least one objective lens that is of known proportions and shapes beforehand by a computer running the algorithm. Once an image is made of this object by the camera, the unscrambling method or algorithm will puzzle back together the scrambled output of the image sensor with respect to a known reference.

In particular, the fibers at the input side are locally sorted to one of the two or more fiber bundle exits at the output side according to a Bayer filter-like structure or pattern, wherein in particular the fibers are equally distributed in a x-direction and a y-direction of an image of an object.

Preferably, the fibers at the input side are randomly sorted or arranged into one of the two or more fiber bundle exits at the output side.

According to another embodiment, the optical system comprises a processing unit for performing an unscrambling algorithm of images at the output side, which are captured by at least two fiber bundle exits, wherein in particular the images are each captured by an image sensor.

In another aspect the invention, the optical system is characterized in that for the unscrambling algorithm line-scan images, in particular in the vertical and horizontal direction, are provided, wherein in particular two-dimensional coordinate sets in the vertical and horizontal direction are used to provide a coordinate map or a look up table (LUT), in particular by means of a script, or that for the unscrambling algorithm static gradient images in the vertical direction and in the horizontal direction, in particular from white to black, are provided, wherein in particular two-dimensional coordinate sets in the vertical and horizontal direction are used to provide a coordinate map or a look up table (LUT), in particular by means of a script.

An exemplary method and algorithm for the multispectral and/or hyperspectral camera (as an optical system) with a random fiber bundle (as optical unit) is configured to represent the object field of the lens with line-scan images, and taking lots of images of an object while scanning it vertically and horizontally. As the location of the line in the object is known while scanning, the location data and the resulting images having travelled through the scrambled fiber bundle are both known. Connecting both these two dimensional special coordinate sets with a script can provide a coordinate map or a look up table (LUT).

Another exemplary method and algorithm for the multispectral and/or hyperspectral camera with the random fiber bundle is configured not to use line-scan images, but two static gradient images from white to black, one vertically (y-direction) and one horizontally (x-direction). As the location of the gradient step in the object is known, the location data and the resulting images having travelled through the scrambled fiber bundle are both known. Connecting both these two dimensional spatial coordinate sets with a script can provide a coordinate map or a look up table (LUT).

In addition, the optical system is characterized in that for the unscrambling algorithm a convolutional neural network (CNN) is used, wherein in particular the convolutional neural network (CNN) is trained with scrambled images, scrambled at the output side of the optical unit, as input values or wherein the convolutional neural network (CNN) is trained by means of a coordinate map or a look up table (LUT) between captured images and original images.

Within the scope of the invention, a convolutional neural network (CNN) can be trained to unscramble the scrambled images of the multispectral and/or hyperspectral camera having a random fiber bundle. This is done by giving the convolutional neural network (CNN) image targets that the camera is also looking at ground truth values, and the respective scrambled images through the randomised fiber bundle of the camera as input values. The output would be the unscrambled images, and with a large enough training set be able to unscramble new and unknown images after the completion of training.

A slightly different way a convolutional neural network (CNN) could be used for unscrambling the scrambled images of the multispectral and/or hyperspectral camera with the random fiber bundle is to train it to make a coordinate map or look up table (LUT) between input mages and original images. Similar to the above embodiment, training the convolutional neural network (CNN) is done by giving it image targets that the camera will also be looking at as ground truth values, and the respective scrambled images through the randomised fiber bundle of the camera as input values. The output would be a look up table (LUT) that during training attempts to unscramble the fiber bundle scrambled images and checks with the target ground truths. The convolutional neural network (CNN) also can be trained on the basis of this check. After a training, a look up table (LUT) is provided or exists, so that a scrambled image can be put in and outputs an unscrambled image.

According to another embodiment, the optical system can be configured as a projector, in particular multi-color projector. In this case, the optical unit is configured as a fiber bundle combiner, which comprises at least two two or more fiber bundle entrances and one fiber bundle exit. For the use of the multi-color projector, one or more light sources, in particular of any kind of spectrum, polarization and intensity, in combination with two or more fiber bundle entrances or fiber bundle input surfaces, either grouped one to one, or any other combination or arrangement, the projector transmits light. The light transmittal can be done directly, with mirrors, lenses, or any kind of light medium. Before the light is entering the fiber bundle beam combiner, it first turns into an image with for example a liquid crystal display (LCD). Once the light, which can be an image, has entered the fiber bundle combiner at the input side, the combiner will combine the light of all the sources into the one output. Each fiber collects the localised light of the display image (e.g. LCD) and transmit it to the fiber bundle exits. In this example of the optical system, the fibers at the fiber bundle entrances are e.g. locally sorted to the fiber bundle exits, similar to how a Bayer filter might sort or structure the filter pattern, equally distributed in the x-direction and y-direction of the image. Hereby, the spatial continuity of the scene and of the image are maintained. After the output at the fiber bundle exit, the fiber bundle combiner can be accompanied by a projection lens of a projector system or the like, which projects the images through the lens, which in turn creates the projection on a screen and/or another display surface.

Within the scope of the invention, the optical unit, which can be provided as a fiber bundle beam splitter and/or combiner, can also be incorporated into other optical or imaging devices, such as but not limited to: open surgery medical camera, fluorescence guided image surgery camera, minimally invasive surgery camera, endoscope, polarizing camera utilising polarising filters, auto-fluorescence camera, hyperspectral camera with spectral reconstruction network (SRNet) and broadband multispectral filter array and/or metrology devices and/or any combination of these exemplary devices.

Moreover, the optical system can be provided for a surgical method for endoscopic surgery and/or for laparoscopic surgery or the like, applying the method for capturing images as described above, preferably by means of a surgical instrument or of an image capturing system or optical system as explained above.

Preferably, the surfaces of the fibers, in particular the end surfaces, of the optical unit are provided with at least one coating, wherein in particular coatings are provided as polarizing coating and or diffraction coating, dielectric interference coating, dichroic coating, metallic coating, index matching coating, antireflective coating, semi-transparent metallic mirror coating, birefrigerant material, liquid crystal polymer air, absorption, retarder and/or magneto-optical coating. Within the scope of the invention, the (end) surfaces of fibers of the optical unit, in particular as a fiber bundle beam splitter and/or combiner, can be modified with any type of multiple coatings, in any combination. The modification of the end surfaces of the fibers can result in a multispectral and/or hyperspectral camera (as mentioned above) or a polarizing camera and an auto-fluorescence camera. In this context, the optical filters mentioned above may be provided, but are not limited to.

According to another preferred embodiment, the optical unit comprises at least one band width unit, wherein in particular the at least one band width unit is provided as a Far ultraviolet (FIR) bandwidth unit, middle ultraviolet (MUV) bandwidth unit, near ultraviolet (NUV) bandwidth unit, visible (VIS) bandwidth unit, near infrared (NIR) bandwidth unit, shortwave infrared (SIR) bandwidth unit, midwave infrared (MWIR) bandwidth unit, long infrared (LIR) bandwidth unit and/or far infrared (FIR) bandwidth unit.

In particular, the arranged fibers of the optical unit, as such a bundle beam splitter and/or combiner, can have multiple bundle cross-section shapes, also known as packing density. For example, the fiber bundle or fiber arrangement can be in either a hexagonal shape, or any value of quality less than hexagonal. Within the scope of the invention, a rectangular shape of the fiber arrangement is also possible.

Preferably, the fibers of the optical unit comprise single mode fibers and/or multimode fibers/or gradient index fibers and/or photonic crystal fibers and/or that the fibers of the optical unit are made out of silica glass, fluoride glass, chalcogenide glass, borosilicate glass, generate glass, plastic polymer, silicon, palladium silver (Pd-Ag) alloy and/or ceramic. The mentioned materials for the fibers can be used for the manufacturing of the fibers. Other and not mentioned suitable materials are also possible.

According to another aspect, the optical system is characterized in that the fiber ends at the input side and/or the fiber ends of the output side are provided as flat surfaces, spherical caps, conical surfaces, elliptic cones, cylindrical surfaces, paraboloid or elliptic paraboloid surfaces, hyperboloid surfaces, Bèzier surfaces, Non-uniform Rationale B-Spline surfaces (NURBS), ellipsoid segment surfaces and/or convex surfaces. Within the context of the invention, the form of the fiber ends of the fibers are not limited to the mentioned surfaces.

Furthermore, in an embodiment of the optical system, lenses or micro-lenses to the ends of the optical fibers at the input side and/or the output side can be provided, in particular for gathering light or for spreading light out.

The object is further solved by a use of an optical system, as described above or according to any of the claims 1 to 13, for a multi-spectral and/or hyperspectral camera of a surgical device, in particular a surgical instrument, or for a, in particular multi-color, projector or for an image capturing system. In order to avoid reputation, reference is expressly made to the above explanations.

In addition, the object is also solved by a method for operating an optical system, as described above or according to any of the claims 1 to 13, wherein the optical system is operated in a multispectral and/or hyperspectral camera of a surgical device, in particular a surgical instrument, or for a, in particular multi-color, projector or for an image capturing system. Reference is also expressly made to the above explanations.

Within the scope of the invention a surgical instrument, in particular endoscope is provided, having an image capturing system (as an optical system) with an optical device (as an optical unit) for capturing images of an area of interest of an object, wherein the optical device has an image splitter with at least two fiber bundles or fiber bundle exits at the output side, wherein the image splitter is configured to separate an incoming image (at the input side) into two or more split images or into the fiber bundles or fiber bundle exits (at the output side).

In this case, an incoming image of an object is separated into at least two (partial) images by means of the image splitter (as optical unit) of the optical device, wherein the split images are transmitted from the input side of the fiber bundle(s) or the fiber bundle entrance(s) at the input side to each output side or output end of the fiber bundles or fiber bundle exits at the output side. The optical device has a fiber-based image splitter (or fiber bundle splitter) in order to separate one incoming image of an object into two or more split or partial images at the output side. The split images are transmitted through the fiber bundles to the output side of the fiber bundles or fiber bundle exits, wherein the transmitted split or partial images are recorded by for example a camera or image sensor or the like. In the fiber bundle (as an optical unit), the split image is effectively separated into discrete pixel-like values by each fiber. Each fiber projects each "pixel" or a pixel-like sensor or sensitive device, which are positioned at the output ends or output side of the fibers or the fiber bundle exits in order to create or generate an image of the split images, transmitted by the fibers of the fiber bundles (as an optical unit). The fibers of the fiber bundles have optical properties to transmit electromagnetic waves in the non-visible and the visible ranges. For example, the fibers of the fiber bundles or the fiber bundle splitter can be glass fibers or plastic fibers.

According to an advantageous embodiment of the surgical instrument, each fiber bundle is configured to transmit the split image from a front side of the fiber bundle at the input side to an output side of the fiber bundle or optical unit.

Furthermore, the surgical instrument is characterized in that a focusing lens, having a focusing plane, is arranged in front of a, in particular flat, front of the image splitter at the input side, wherein the front of the image splitter is arranged in the focusing plane of the focusing lens. In particular, the focusing plane of the focusing lens and the flat front of the image splitter are parallel to each other. Preferably, the flat input ends or input sides of the fiber bundles are arranged in the flat front of the image splitter. According to a further embodiment, the output ends of the fiber bundles are also provided as flat output ends at the output side.

In a preferred embodiment of the surgical instrument, image sensors are each arranged at the output side of the fiber bundles or fiber bundle exits, wherein each image sensor is configured to capture the split image transmitted by the fiber bundle (or optical unit). The image sensors can be CMOS sensors or sensitive plates, which are arranged adjacent to the output side of the fiber bundles.

Preferably, an optical coating is provided on at least one output side of the fiber bundles. By means of an optical coating or optical coatings on the output side of the fiber bundles, it is possible to create a multi-spectral image splitter for the image capturing system of the surgical instrument.

According to a preferred embodiment of the surgical instrument, different optical coatings are provided on the output side of at least two or all fiber bundles.

Besides, it is preferred in a further embodiment of the surgical instrument that the fiber bundles have an input side for the split image, wherein the input sides of the fiber bundles are arranged in one plane.

The surgical instrument is further preferably characterized in that the fiber bundles have cross-sections, wherein the cross-sections of the fiber bundles are arranged at the input side and at the output side in a predetermined order in order to capture the split images directly.

In a further embodiment of the surgical instrument, the fiber bundles have randomized fibers, wherein the captured split images at the output side of the fiber bundles are or will be, preferably digitally, un-randomized, wherein for the randomization of captured images an unrandomizing algorithm (as described above) can be executed by a processing device, like a computer.

Furthermore, the disclosure of this application provides also a use of an image capturing system with an optical device for capturing images of an area of interest of an object in a surgical instrument, in particular endoscope, as described above. In order to avoid repetitions, reference is expressly made to the above explanations and embodiments.

Besides, within the disclosure of the application a method for operating a surgical instrument, in particular endoscope, is provided as described above. In particular, the method is used or performed for endoscopic surgery, wherein the surgical instrument is operated in order to capture images of an object of interest during the surgery.

Finally, within the disclosure of the application a surgical method for endoscopic surgery and/or for laparoscopic surgery is provided, applying the method for capturing images as described above, preferably by means of a surgical instrument or of an image capturing system or of an optical system or optical unit as explained above.

Further characteristics of the invention will become apparent from the description of the embodiments according to the invention together with the claims and the included drawings. Embodiments according to the invention can fulfill individual characteristics or a combination of several characteristics.

The invention is described below, without restricting the general intent of the invention, based on exemplary embodiments, wherein reference is made expressly to the drawings with regard to the disclosure of all details according to the invention that are not explained in greater detail in the text. The drawings show in:
- Fig. 1: a schematic representation of an endoscope according to the prior art,
- Fig. 2: schematically examples for fiber bundles at an input side or an output side of an optical unit for a surgical device,
- Fig. 3: schematically an example of a fiber bundle splitter,
- Fig. 4: an schematic example a fiber bundle combiner,
- Fig. 5: an embodiment of a fiber bundle splitter and combiner,
- Fig. 6: an exemplary illustration of a multispectral/hyperspectral camera,
- Fig. 7: schematically an example of fiber bundles,
- Fig. 8: schematically the transmission of different target inputs at different time steps at the output side of a fiber bundle exit.
- Fig. 9: an schematic embodiment of a projector,
- Fig. 10: three exemplary examples of cross-sections of fiber bundle entrances and/or fiber bundle exits,
- Fig. 11a: schematically an embodiment of an optical device of an endoscope in section and
- Fig. 11b: schematically another embodiment of an optical device of an endoscope in section.

In the drawings, the same or similar types of elements or respectively corresponding parts are provided with the same reference numbers in order to prevent the item from needing to be reintroduced.

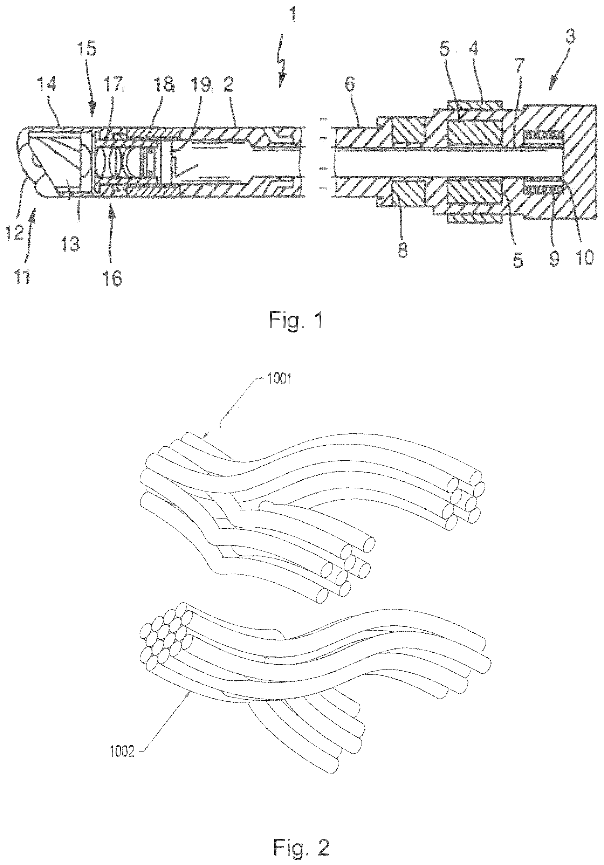

In Fig. 1 an endoscope 1 known from the prior art is shown schematically. The endoscope 1 has at the proximal end, shown to the right, a grip 3, which leads into an endoscope shaft 2. The distal end of the endoscope shaft 2 is shown on the left-hand side in Fig. 1.

The grip 3 has a rotary ring 4, the inner tube 7 being able to be rotated thereby relative to an outer tube 6 via bar magnets 5, which are connected to the inner tube 7, in order to alter the viewing direction of the endoscope 1. The inner tube 7 is also mounted in the grip 3 by means of a radial bearing 8. Moreover, the grip 3 has a pretensioning apparatus consisting of a compression spring 9, which is pretensioned relative to a stop 10 for the compression spring 9. The compression spring 9 ensures that the inner tube 7 is pressed and/or pretensioned in the axial direction toward the distal end 11 of the endoscope shaft 2.

At the distal end 11 the endoscope shaft 2 has an aperture 12 which faces laterally. An optical assembly 13 with lenses and prisms is located behind the aperture 12, the light entering through the aperture 12 being deflected thereby in a direction parallel to the longitudinal axis of the endoscope shaft 2. The optical assembly 13 is mounted by a mounting 14, which is connected to the outer tube 6. The aperture 12 is also part of the optical assembly 13.

Proximally, a second optical assembly 16 adjoins the first optical assembly 13, said second optical assembly in this case terminating in an image sensor unit 19. The second optical assembly 16 is mounted in a mounting 17 which is connected to the inner tube 7 such that it performs rotations or displacements of the inner tube 7 therewith. The inner tube 7 is mounted radially relative to the outer tube 6 in the region of the distal end 11 of the endoscope shaft 2, by means of a radial bearing 18.

The distal front surface of the mounting 17 of the second optical assembly 16 and the proximal front surface of the mounting 14 of the first optical assembly 13 are arranged opposite one another and form an axial bearing 15. By the pretensioning of the inner tube 7 in the axial direction by the compression spring 9 in the grip 3 the axial bearing 15 is closed, i.e. the distal front surface of the mounting 17 is pressed against the proximal front surface of the mounting 14. As a result, the axial position of the second optical assembly 16 is fixedly defined relative to the first optical assembly 13.

Fig. 2 shows exemplary the basics of any fiber bundle for an optical unit for a surgical device. The upper picture of the fiber bundle shows a fiber bundle entrance 1001 of a fiber bundle splitter at an input side. The fiber bundle entrance 1001 comprises a plurality of individual fibers at the input side, which are divided into several bundles of fibers for the fiber bundle exits at the output side.

The bottom picture of Fig. 2 illustrates schematically a fiber bundle exit 1002 of a fiber bundle combiner at the output side. The fiber bundle exit 1002 comprises a plurality of individual fibers at the output side of the fiber bundle combiner.

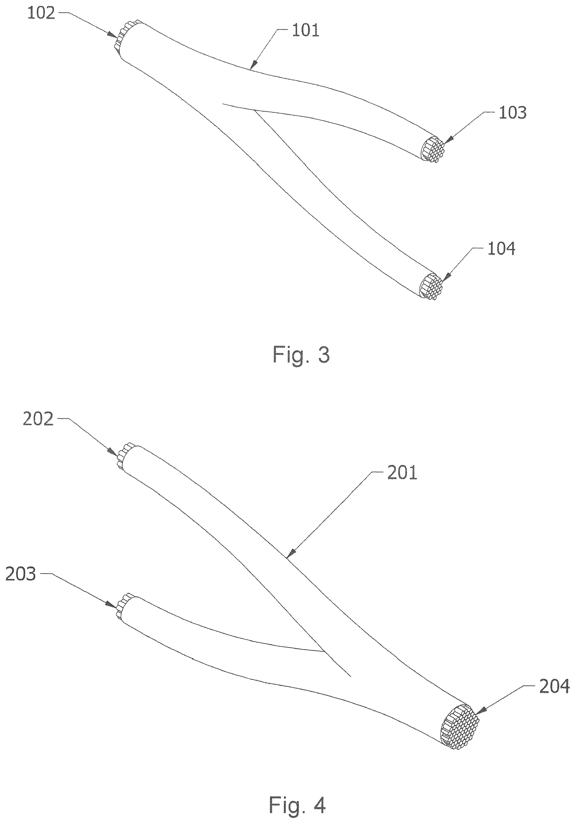

Fig. 3 illustrates schematically an example of a fiber bundle splitter 101 comprising a single fiber bundle entrance 102 (at an input side) and two fiber bundle exits 103 and 104 (at an output side).

Fig. 4 shows an example a fiber bundle combiner 201 comprising two fiber bundle entrances 202, 203 (at an input side) and a combined fiber bundle exit 204 (at an opposite side).

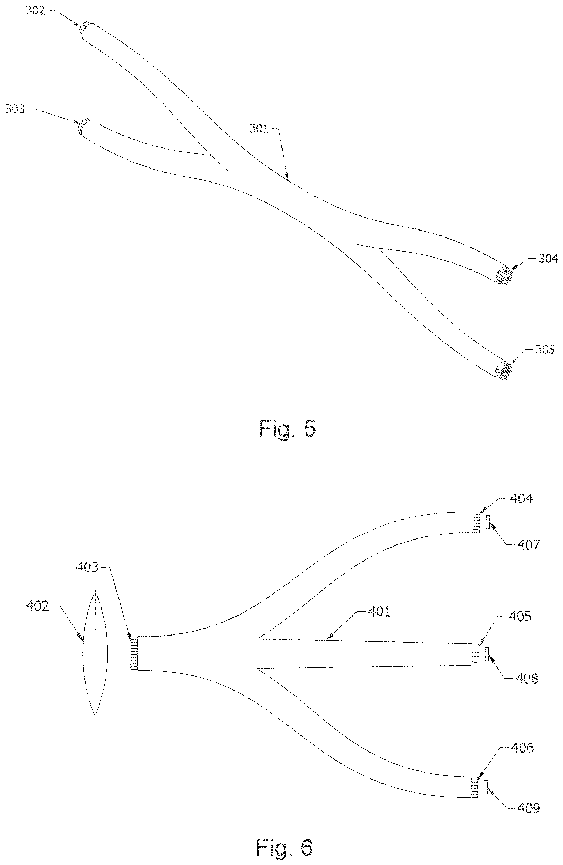

Fig. 5 shows an example of a fiber bundle splitter and combiner 301 comprising two fiber bundle entrances 302, 303 (at an input side) and split fiber bundle exits 304 and 305 (at an output side).

Fig. 6 shows an example of a multispectral/hyperspectral camera having a fiber bundle splitter 401 to split and transmit the images. An objective imaging lens 402 transmits an image onto an fiber bundle entrance 403 (at an input side). The fiber bundle splitter 401 transmits the captured and split image to the fiber bundle exits 404, 405 and 406 (at an output side). Sensors 407, 408 and 409 for the fiber bundle exits 404, 405 and 406 capture the split images.

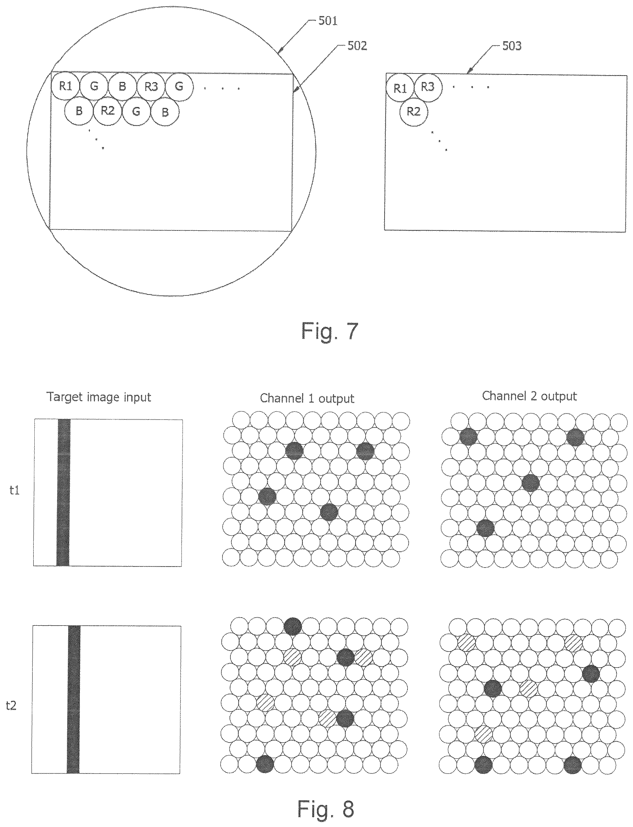

Fig. 7 illustrates an example of how to locally sort the fiber bundles from entrance to exit. The parts 501, 502 of an image (on the left side) are a cross-section view of the fiber bundle entrance (at an input side) with a plurality of individual fibers. The right part 503 shows a cross-section view of the fiber bundle exit (at an output side). The part 501 is the blackening of the objective lens or field of view limit in an example use case such as the multispectral/hyperspectral camera, and the part 502 is the effective image size of the sensors used in the same example. R designates fiber cross-sections that go to one channel, G likewise and also B. The output of R is represented on the right side at 503, and shows its sorting.

Fig. 8 shows for different target inputs at different time steps t1 and t2 and the parallel output of the transmitted image for the two channels 1 and 2. t1 and t2 are time steps between themselves, if looking at the target image input, the vertical line-scan bar moves slightly to the right. This results in different random or scrambled fibers on both split fiber bundle channel 1 and channel 2 to occur. In t2 on both channel outputs, the previous lit fibers of t1 have been shaded slightly to show each fiber will uniquely light up per time step.

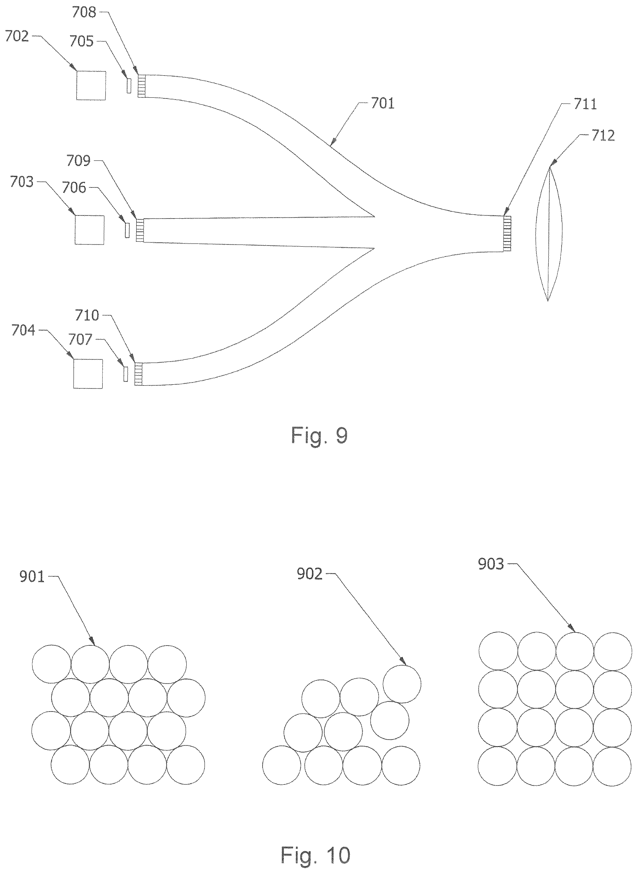

Fig. 9 shows an example of a projector 701 having a fiber bundle combiner. Light of the light sources 702, 703 and 704 (at an input side) are coupled via liquid crystal displays (LCDs) 705, 706 and 707 into fiber bundle entrances 708, 709, 710 of the projector 701. The transmitted combined light exits the projector 701 at the common fiber bundle exit 711 (at an output side). A lens 712 at the fiber bundle exit 711 focusses the exiting light onto a target or the like.

Fig. 10 shows three examples of cross-sections of fiber bundle entrances and/or exits of a fiber bundle. The left example 901 shows a perfect hexagonal formation of the fiber ends or fibers, while the middle example 902 shows a more random formation of the fibers and the right example 903 shows a rectangular formation of the fibers.

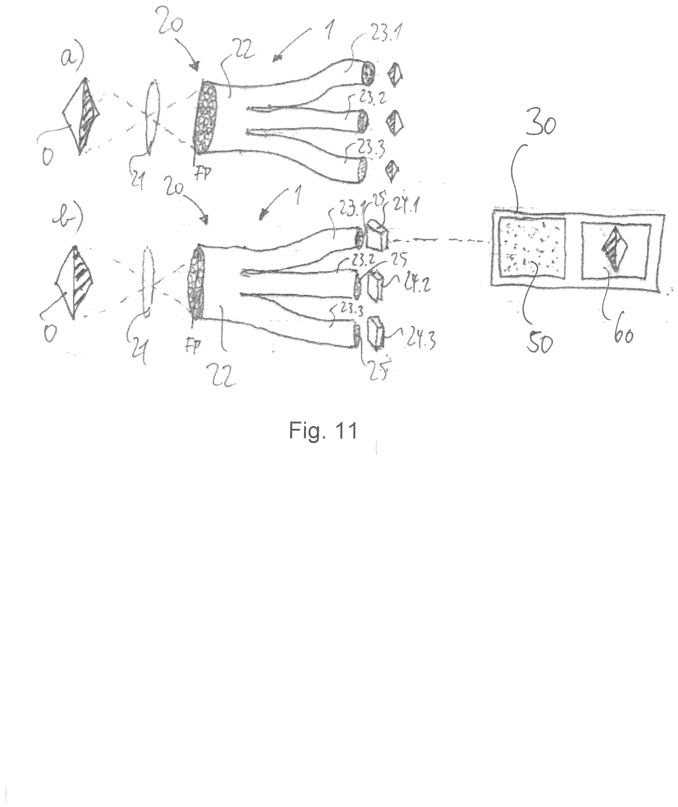

Fig.11a and 11b show exemplary illustration of embodiments of an optical device 20 of an endoscope 1. The optical device 20 comprises a focusing lens 21 with a focusing plane FP. Furthermore, the optical device 20 comprises a fiber image splitter 22, wherein the fiber image splitter 22 comprises several fiber bundles 23.1, 23.2, 23.3 each consisting of a bundle of glass fibers or the like.

The input side of the image splitter 22 is arranged in the focusing plane FP of the focusing lens 21. The focusing lens 21 is part of an imaging section (not shown) including an objective optical system, wherein the focusing lens 21 forms or projects an image of an object O on the input end or input side of the image splitter 22. The image splitter 22 is configured to separate the incoming image of the object O, wherein the split images of the object O are transmitted by means of the fibers of the fiber bundles 23.1, 23.2, 23.3 from the input side to the output side of the fiber bundles.

According to the embodiment in Fig. 2b, three image sensors 24.1, 24.2, 24.3 are positioned at the output end of the fiber bundles 23.1, 23.2, 23.3 in order to capture the transmitted split and randomized images. For example, the image sensors 24.1, 24.2, 24.3 can be CMOS image sensors or the like.

According to a further embodiment of the image splitter 22, the output side or output end of the fiber bundles 23.1, 23.2, 23.3 can be coated with, in particular different, coatings 25, in order to provide a multi-spectral image splitter, wherein the coatings have different transmittances for the wavelength of the split images, transmitted by the fiber bundles 23.1, 23.2, 23.3.

The fiber bundles 23.1, 23.2, 23.3 at the output side in the embodiment of Fig. 2a are non-randomized fiber bundles, wherein the cross-sections of the fiber bundles 23.1, 23.2, 23.3 are ordered in such a way that the protections of the transmitted split images at the output ends can be directly seen. In case of randomized fiber bundles 23.1, 23.2, 23.3 (see Fig. 2b), no recognizable images can be seen of any of the output ends of the fiber bundles 23.1, 23.2, 23.3, as these captured images are randomized. The captured randomized images are transferred to a processing device 30, which is in particular provided as a computer or the like. The processing device 30 receives the randomized images 50, which are further unrandomized by a randomizing algorithm, which is executed on the processing device 30. After the digital unrandomizing process of the randomized images, the un-randomized images 60 can be displayed.

All named characteristics, including those taken from the drawings alone, and individual characteristics, which are disclosed in combination with other characteristics, are considered alone and in combination as important to the invention. Embodiments according to the invention can be fulfilled through individual characteristics or a combination of several characteristics. Features which are combined with the wording "in particular" or "especially" are to be treated as preferred embodiments.

### List of reference numbers

- 1: endoscope
- 2: shaft
- 3: grip
- 4: rotary ring
- 5: bar magnet
- 6: outer tube
- 7: inner tube
- 8: radial bearing
- 9: compression spring
- 10: stop for compression spring
- 11: distal end
- 12: aperture
- 13: first optical assembly with lenses and prisms
- 14: proximal front surface of the mounting
- 15: axial bearing
- 16: second optical assembly
- 17: distal front surface of the mounting
- 18: radial bearing
- 19: image sensor unit
- 20: optical device
- 21: focusing lens
- 22: image splitter
- 23.1, 23.2, 23.3: fiber bundle
- 24.1, 24.2, 24.3: image sensor
- 25: coating
- 30: processing device
- 50: randomized image
- 60: randomized image
- 101: fiber bundle splitter
- 102: fiber bundle entrance
- 103, 104: fiber bundle exit
- 201: fiber bundle combiner
- 202, 203: fiber bundle entrance
- 204: fiber bundle exit
- 301: fiber bundle splitter and combiner
- 302, 303: fiber bundle entrance
- 304, 305: fiber bundle exit
- 401: fiber bundle splitter
- 402: objective imaging lens
- 403: fiber bundle entrance
- 404, 405, 406: fiber bundle exit
- 407, 408, 409: sensor
- 501: part
- 502: part
- 503: part
- 701: projector
- 702, 703, 704: light source
- 705, 706, 707: liquid crystal display
- 708, 709, 710: fiber bundle entrance
- 711: fiber bundle exit
- 712: lens
- 901: hexagonal formation
- 902: random formation
- 903: rectangular formation
- 1001: fiber bundle entrance
- 1002: fiber bundle exit

- FP: focusing plane
- O: object
- t1, t2: time step

## Claims

1. An optical system for a surgical device, in particular a surgical instrument, comprising an optical unit having an optical fiber bundle with an input side and an output side, wherein the input side has at least one or more fiber bundle entrances and the output side has at least one or more fiber bundle exits, wherein the sum of the number of the fiber bundle entrances and of the number of the fiber bundle exits is greater than 2.

2. The optical system according to claim 1, **characterized in that** the optical unit is configured as a fiber bundle beam splitter or as a fiber bundle beam combiner or as a bundle beam splitter and combiner.

3. The optical system according to claim 1 or 2, **characterized in that** the optical fiber bundle of the optical unit has an amount of a predetermined number n of individual fibers, wherein the predetermined number n of the individual fibers is divided between k (k=1, 2, 3, ...) fiber bundle entrances at the input side and/or wherein the predetermined number n of the individual fibers is divided between l (l=1, 2, 3, ...) fiber bundle exits at the output side.

4. The optical system according to any of the claims 1 to 3, **characterized in that** the optical system is configured as a multi-spectral and/or hyperspectral camera, wherein in particular at least one objective lens is provided in front of the input side for projecting an image onto the at least one fiber bundle entrance.

5. The optical system according to any of the claims 1 to 4, **characterized in that** the fibers at the input side are locally sorted to one of the two or more fiber bundle exits at the output side according to a Bayer filter-like structure or pattern, wherein in particular the fibers are equally distributed in a x-direction and a y-direction of an image of an object.

6. The optical system according to any of the claims 1 to 4, **characterized in that** fibers at the input side are randomly sorted or arranged into one of the two or more fiber bundle exits at the output side.

7. The optical system according to any of the claims 1 to 6, **characterized in that** the optical system comprises a processing unit for performing an unscrambling algorithm of images at the output side, which are captured at least two fiber bundle exits, wherein in particular the images are each captured by an image sensor.

8. The optical system according to claim 7, **characterized in that** for the unscrambling algorithm line-scan images, in particular in the vertical and horizontal direction, are provided, wherein in particular two-dimensional coordinate sets in the vertical and horizontal direction are used to provide a coordinate map or a look up table (LUT), in particular by means of a script, or that for the unscrambling algorithm static gradient images in the vertical direction and in the horizontal direction, in particular from white to black, are provided, wherein in particular two-dimensional coordinate sets in the vertical and horizontal direction are used to provide a coordinate map or a look up table (LUT), in particular by means of a script.

9. The optical system according to claim 7 or 8, **characterized in that** for the unscrambling algorithm a convolutional neural network (CNN) is used, wherein in particular the convolutional neural network (CNN) is trained with scrambled images, scrambled at the output side of the optical unit, as input values or wherein the convolutional neural network (CNN) is trained by means of a coordinate map or a look up table (LUT) between captured images and original images.

10. The optical system according to any of the claims 1 to 9, **characterized in that** the surfaces of the fibers, in particular the end surfaces, of the optical unit are provided with at least one coating, wherein in particular coatings are provided as polarizing coating and or diffraction coating, dielectric interference coating, dichroic coating, metallic coating, index matching coating, antireflective coating, semi-transparent metallic mirror coating, birefrigerant material, liquid crystal polymer air, absorption, retarder and/or magneto-optical coating.

11. The optical system according to any of the claims 1 to 10, **characterized in that** the optical unit comprises at least one band width unit, wherein in particular the at least one band width unit is provided as a Far ultraviolet (FIR) bandwidth unit, middle ultraviolet (MUV) bandwidth unit, near ultraviolet (NUV) bandwidth unit, visible (VIS) bandwidth unit, near infrared (NIR) bandwidth unit, shortwave infrared (SIR) bandwidth unit, midwave infrared (MWIR) bandwidth unit, long infrared (LIR) bandwidth unit and/or far infrared (FIR)bandwidth unit.

12. The optical system according to any one of the claims 1 to 11, **characterized in that** the fibers of the optical unit comprise single mode fibers and/or multimode fibers/or gradient index fibers and/or photonic crystal fibers and/or that the fibers of the optical unit are made out of silica glass, fluoride glass, chalcogenide glass, borosilicate glass, generate glass, plastic polymer, silicon, palladium Silver alloy and/or ceramic.

13. The optical system according to any of the claims 1 to 12, **characterized in that** the fiber ends at the input side and/or the fiber ends of the output side are provided as flat surfaces, spherical caps, conical surfaces, elliptic cones, cylindrical surfaces, paraboloid or elliptic paraboloid surfaces, hyperboloid surfaces, Bèzier surfaces, Non-uniform Rationale B- spline surfaces, ellipsoid segment surfaces and/or convex surfaces.

14. A use of an optical system according to any of the claims 1 to 13 for a multispectral and/or hyperspectral camera of a surgical device, in particular a surgical instrument, or for a, in particular multi-color, projector or for an image capturing system.

15. A method for operating an optical system according to any of the claims 1 to 13, wherein the optical system is operated in a multispectral and/or hyperspectral camera of a surgical device, in particular a surgical instrument, or for a, in particular multi-color, projector or for an image capturing system.
